# EUROPEAN PATENT SPECIFICATION

(11) **EP 1 621 227 B1**
(45) Date of publication and mention of the grant of the patent: **14.03.2012**
(21) Application number: 04729221.4
(22) Date of filing: 23.04.2004
(51) Int. Cl.: A61N 5/067

(54) **CIRCULATION PROMOTING LASER IRRADIATION DEVICE**
KREISLAUFFÖRDERNDE LASER-BESTRAHLUNGSVORRICHTUNG
DISPOSITIF D'IRRADIATION PAR LASER FAVORISANT LA CIRCULATION

(30) Priority: 06.05.2003 JP 2003127903
(43) Date of publication of application: 01.02.2006
(73) Proprietor: Terumo Kabushiki Kaisha, Tokyo 151-0072 (JP)
(72) Inventor: Ishii, Takeo, Ashigarakami-gun, Kanagawa 259-0151 (JP); Horiuchi, Kunio, 139 Inume-machi, Hachioji-shi, Tokyo 193-0802 (JP)
(74) Representative: Casalonga, Axel
(86) International application number: PCT/JP2004/005851
(87) International publication number: WO 2004/105876

(56) References cited:
- EP-A- 0 947 221
- EP-A- 1 238 683
- WO-A-98/43703
- JP-A- 1 064 674
- JP-A- 1 064 674
- JP-A- 1 136 668
- JP-A- 11 276 499
- JP-A- 60 114 273
- JP-A- 2001 187 157
- US-A1- 2001 007 080

## Description

### Technical Field

The present invention relates to a circulation-accelerating laser irradiation system, particularly to a circulation-accelerating laser irradiation system by which laser beams are concentratedly radiated onto a subcutaneous target part from positions over a skin.

In particular the present invention relates to a blood circulation - accelerating system according to the preamble of claim 1 such as it is for example known from US 2001/007080 A1 or WO 98/43703A.

### Background Art

In recent years, in the regions of pain clinics and orthopaedics, ray therapeutic apparatuses such as low reaction level laser therapeutic apparatuses and linear polarized near infrared ray therapeutic apparatuses have been utilized as a therapeutic means for a focus present at a subcutaneous deep part in the cases of stiff shoulders, lumbago and the like. Examples of such ray therapeutic apparatuses include a system for irradiating a therapeutic target part with laser beams (Japanese Patent Laid-open No. 2000-187157) and a system for irradiating a therapeutic target part with monochromatic light (Japanese Patent Laid-open No. 2001-212250).

Generally, these ray therapeutic apparatuses are known as a means for alleviating a stiffness or ache by irradiating the target part with light having various wavelengths from positions over the skin.

As an action of the ray therapy using the ray therapeutic apparatus, a nerve conduction blocking effect has been known. On the other hand, diffusion or removal of pain-related substances (bradykinin, histamine, prostaglandin, etc.) and fatigue-related substances (lactic acid, etc.) from a local part through improvement of circulation is regarded as important.

In addition, a direct relaxing effect on blood vessel smooth muscle has come to be known as a principal mechanism of the circulation improving effect.

Besides, it has been reported that rays on the short wavelength side are effective for enhancing the effect of light. It is described, for example, in such references as Furchgott et al. (J. Gen. Physiol. 44:449-519, 1961), Furchgott et al. (J. pharmacol. expe. Ther. 259:1140-1146, 1991) and Matsuno et al. (Laser Med. Sci. 15:181-187, 2000).

The reason for these report lies in that, while the wavelength of the rays used in the conventional ray therapeutic apparatuses is 810-830 nm in the case of laser and 600-1600 nm in the case of linear polarized near infrared rays, it has been found that the circulation-improving (vasodilating or the like) effect constituting one of the analgesic mechanisms is greater on the shorter wavelength side. Particularly, the above three references show that irradiation with ultraviolet rays (300-350 nm) at a very weak output relaxes blood vessels strongly.

Although the conventional ray therapeutic apparatuses are highly evaluated in view of their little side effects, they are pointed out to have problems such as a yet insufficient desired effect and a longer therapeutic period.

Furthermore, for a sufficient quantity of rays to reach a focus at a subcutaneous deep part (blood vessels in fascias or muscles under a subcutaneous fatty tissue in the case of muscle- or fascias-related lumbago) from a position or positions over the skin, it is necessary to irradiate with energy at a comparatively high output. If irradiation with energy is conducted at a too high output, the surface layer part of the skin may be damaged. For example, the ray therapeutic apparatuses in clinical use include those with an output in excess of 1000 mW.

Furthermore, the rays in the UV region of 300-350 nm have a stimulating action harmful to the skin, and are low in tissue depth reaching performance, so that they are considered to be unsuitable for ray therapy in a percutaneous mode. On the other hand, the rays in the higher UV region and the visible region do not have any harmful action on the skin, but they are high in absorption by hemoglobin in the blood and are not good in tissue depth reaching performance, so that it is highly possible that these rays cannot show a therapeutic effect on a practical basis.

The present invention has been made in consideration of the above-mentioned problems. Accordingly, it is an object of the present invention to provide a circulation-accelerating laser irradiation system by which beams with such a high wavelength as to show a vasodilating effect can be efficiently radiated onto an target part constituting a focus part.

### Summary of the Invention

In order to obtain the above - mentioned objects, the invention proposes a blood circulation accelerating laser irradiation system according to claim 1. The dependent claims relate to advantageous embodiments.

### Brief Description of Drawings

Fig. 1 is a schematic diagram showing a blood vessel sample experiment apparatus.
Fig. 2 is a schematic diagram showing a circulation-accelerating laser irradiation system according to a first embodiment of the present invention.
Fig. 3 is a schematic diagram showing a circulation-accelerating laser irradiation system according to a second embodiment of the present invention.
Fig. 4 is a diagram showing laser beam outgoing ports.

### Disclosure of Invention

Now, the present invention will be described in detail below.

Before describing the circulation-accelerating laser irradiation system according to the present invention, first, description will be made of the vasodilating action obtained by irradiation with light having the wavelength which is utilized in the circulation-accelerating laser irradiation system.

Hitherto, it has been known that light with a wavelength in the UV region has a strong action of dilating blood vessels, but little investigation has been made of light with a wavelength in a visible region (400 to 600 nm).

In view of this, the present inventors have made a comparative investigation of the vasodilating action obtained by irradiation with light, using a blood vessel enucleated from a rat, a laser beam with a wavelength of 532 nm as a beam in the visible region, and a laser beam with a wavelength of 810 nm on the longer wavelength side.

As a result, it has been found out that even the laser beam with a wavelength of 532 nm shows a sufficient vasodilating action. The method and results of this exemplary experiment will be shown below.

### [Blood Vessel Sample Experiment]

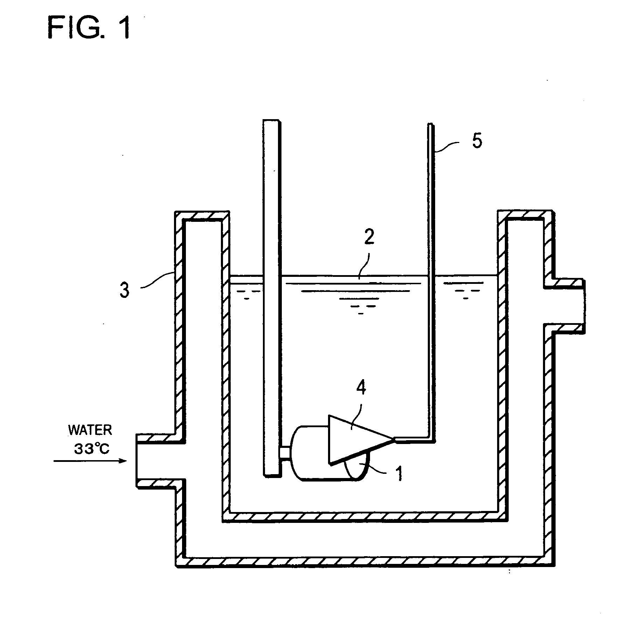

Fig. 1 is a schematic diagram showing a blood vessel sample experiment apparatus.

A rat was used as an animal to be tested, the rat was clubbed to death, followed by phlebotomy, the desending thoracic aorta was enucleated, and a specimen 3 mm long and 1.5 mm in diameter was prepared as a blood vessel sample.

As shown in Fig. 1, the blood vessel sample 1 is suspended in a organ bath 3 charged with 50 ml of Krebs-bicarbonate solution 2, and variation in tension is recorded isometrically. The organ bath 3 is formed of 1 to 2 mm thick glass sheets, and has a double structure so that water can be circulated along an outer peripheral part thereof.

The temperature of the Krebs-bicarbonate solution was so controlled that the inside liquid temperature was 33°C when water at a fixed temperature is circulated in the outer peripheral part of the organ bath 3. A mixed gas of 95% oxygen and 5% carbon dioxide is passed through the inside liquid.

The composition of the Krebs-bicarbonate solution is 118 mM of NaCl, 4.8 mM of KCl, 2.5 mM of CaCl₂, 1.0 mM of MgSO₄, 1.2 mM of KH₂PO₄, 24 mM of NaHCO₃, and 11 mM of glucose.

The enucleated blood vessel has no tension; for checking the relaxation, therefore, the blood vessel was preliminarily contracted with noradrenaline (sympathetic nerve mediator).

Specifically, the blood vessel was contracted by use of 0.03 µM noradrenaline, and after the contraction became constant, the laser irradiation experiment was started.

As the laser irradiation system for a wavelength of 532 nm, there was used KTG Green Laser Irradiation System (output variable to 20 mW) produced by KTG. Co., Ltd.

On the other hand, as the laser irradiation system for a wavelength of 810 nm, there was used a dental semiconductor laser apparatus with an output variable to 100 mW, produced by Yunitaku Co., Ltd.

The laser beam 4 with each individual wavelength was guided by an optical fiber 1.0 mm in diameter, and was radiated directly onto the blood vessel sample 1. The irradiation time per run is 1 minute.

The distance between the blood vessel sample 1 and the tip end of the optical fiber 5 was set to be 1 to 2 mm, to avoid contact therebetween.

The irradiation intensity of laser from the tip end of the optical fiber 5 was measured immediately before irradiation, by use of Field Master FM (produced by COHERENT, USA).

The degree of relaxation of the blood vessel sample 1 was indicated in terms of percentage of noradrenaline contraction, and the results were expressed in terms of mean±SD. The experimental results, obtained at 33°C, are shown in Table 1.

**Table 1**

| Relaxation reactions (at 33°C) with laser beams different in intensity and wavelength | | | | | | |
|---|---|---|---|---|---|---|
| | Relaxation reaction (%) | | | | | |
| | 1mW | 4mW | 10mW | 20mW | 50mW | 100mW |
| 532nm | 41.1±6.0 (n=4) | | | 49.4±10.4 (n=4) | | |
| 810nm | | 4.0±4.1 (n=3) | 10.6±6.7 (n=3) | 12.6±8.7 (n=3) | 26.3±14.8 (n=3) | 5.2 (n=1) |

To the blood vessel sample 1, i.e., the blood vessel (aorta) enucleated from the rat which was provided with a mild tonus by use of noradrenaline, the laser beam with a wavelength of 532 nm produced a comparatively strong relaxation (a relaxation reaction of suppressing the noradrenaline contraction by about 40%) at an intensity of 1 mW, whereas the laser beam with a wavelength of 810 nm produced little reaction at an intensity of 4 to 10 mW and only produced a slight relaxation at an intensity of 50 mW.

In other words, the experimental results verified the usefulness of the laser beam with a visible region wavelength of 532 nm.

When the same experiment was conducted by changing the temperature of the Krebs-bicarbonate solution from 33°C to 36°C, the vasodilating action was greater at 33°C.

Next, the results of an experiment in which an animal was wholly used will be shown below. The method and results of the experiment are as follows.

A rat was used as an animal subjected to the experiment, the rat was anesthetized with pentobarbital, and a probe of a bloodstream measuring instrument (ADVANCE Laser Flowmeter AFL21R produced by ADVANCE Co., Ltd.) was put into close contact with the inside of an auricle part of the rat.

From the outside of the auricle part, the auricle was clamped so that the radiating port of the laser irradiation apparatus is located directly above the probe on the inside.

The bloodstream in the auricle part was recorded on a pen recorder.

The bloodstream immediately upon 1 minute irradiation with laser and that immediately upon 5 minutes irradiation with laser were shown in terms of their increase ratio (mean±SD) based on the bloodstream immediately before the irradiation.

As for the temperature, a temperature measuring probe in place of the probe of the bloodstream measuring instrument was put into close contact with the inside of the auricle part, then irradiation with laser was conducted in the same manner as above, and the temperature was recorded upon 1 minute irradiation, upon 5 minutes irradiation, and upon 10 minutes irradiation. The results are shown in Table 2.

**Table 2**

| Influences of laser on bloodstream in rat auricle part | | | |
|---|---|---|---|
| Wavelength | Intensity | Increase ratio (%) | |
| | | Upon 1 minute irradiation | Upon 5 minute irradiation |
| 532nm | 5mw | 2.0±2.5 | 9.8±10.2 |
| | 20mW | 43.6±34.8 | 95.8±56.6 |
| 810nm | 20mW | 2.8±5.5 | 6.5±13 |
| (n=4 to 5) | | | |

The laser irradiation with a wavelength of 532 nm at an intensity of 5 mW and the laser irradiation with a wavelength of 810 nm at an intensity of 20 mW produced little influence on the bloodstream, whereas the laser irradiation with a wavelength of 532 nm at an intensity of 20 mW produced an increase in the bloodstream in the auricle dependently on time.

Variations in the temperature during the irradiation were also investigated. The results are shown in Table 3.

**Table 3**

| Variations in temperature of rat auricle part by laser irradiation | | | | | |
|---|---|---|---|---|---|
| Wavelength | Intensity | Temperature (°C) | | | |
| | | Before irradiation | Upon 1 minute irradiation | Upon 5 minute irradiation | Upon 10 minute irradiation |
| 532nm | 20mW | 27.4 | 28.9 | 29.8 | 29.8 |
| 810nm | 20mW | 27.2 | 28.9 | 29.5 | 29 |

As seen from the results shown in Table 3, no difference in temperature variation was recognized between the wavelength of 532 nm and the wavelength of 810 nm. Based on the results shown in Tables 2 and 3, therefore, the bloodstream increasing action observed attendant on the irradiation with laser is considered to be an influence of the laser beam itself.

From the above experimental results, it is understood that the irradiation with a laser beam having a wavelength in the visible region has a vasodilating action. The vasodilating action leads to an acceleration of the circulation of blood, whereby diseases caused by hindrance of bloodstream in the fascias at the interface between the skin and the muscle or in the muscle at a subcutaneous deep part, such as muscle- or fascias-relating lumbago and stiff shoulders, can be alleviated.

Since it has been found that the beam having a wavelength of 532 nm and a low energy (e.g., 1 mW) dilates the blood vessel strongly as above-described, a therapeutic effect can be expected when that much energy is made to reach the fascias present at the interface between the skin and the muscle at a subcutaneous deep part.

However, the energy with a wavelength of 532 nm is b liable to be absorbed by hemoglobin and the like, and is therefore poor in tissue depth reaching performance. For permitting an effective amount of the laser beam with the wavelength to reach a target part at a subcutaneous deep part, a high energy is needed. On the other hand, the use of only one light source may result in that the irradiated part of the skin is damaged by heat.

In view of these points, in the present invention, beams with little irradiation energy are radiated from a plurality of directions, whereby the optical energy per beam is reduced, the influence of each individual beam on the skin is thereby reduced, and the plurality of irradiation beams are concentrated onto the target part at a subcutaneous deep part, whereby a sufficient optical energy is supplied to the target part.

### [First Embodiment]

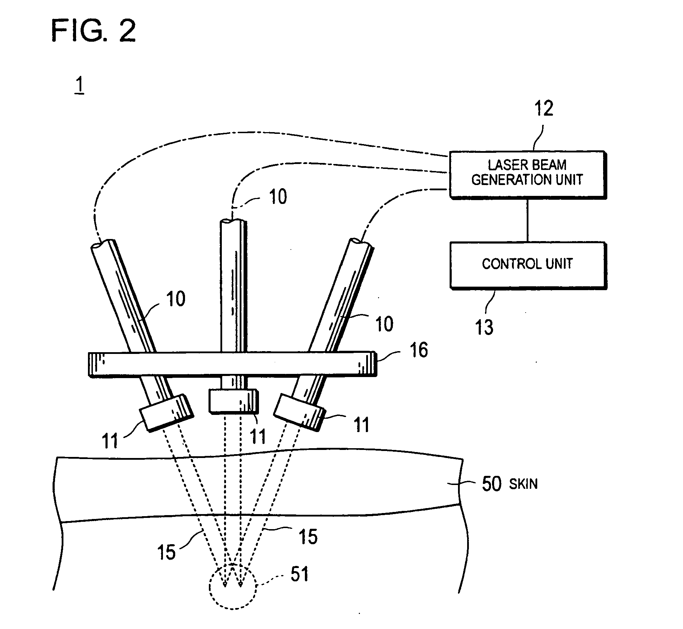

Fig. 2 is a schematic diagram showing a circulation-accelerating laser irradiation system according to a first embodiment of the present invention.

The circulation-accelerating laser irradiation system 1 according to the first embodiment has a plurality of laser irradiation units by which laser beams with such a wavelength as to have a vasodilating action are radiated as parallel beams from a plurality of different directions over a skin, and a concentrating device for concentrating the plurality of laser beams radiated from the plurality of laser irradiation units onto a subcutaneous target part.

The laser irradiation unit includes a plurality of optical fibers 10, and a plurality of collimator lenses 11 attached to the tip ends of the optical fibers 10. The other ends of the optical fibers 10 are connected to a laser beam generation unit 12. The laser beam generation unit 12 can supply laser beams respectively into the plurality of optical fibers 10. The laser beam generation unit 12 is further connected to a control unit 13.

The plurality of optical fibers 10 provided with the collimator lenses 11 at their tip ends are fixed by a holding member 16. The holding member 16 functions as a concentrating device, and position the optical fibers 10 so that laser beams 15 radiated from the optical fibers 10 are concentrated onto a target part (focus part) 51. This positioning ensures that the laser beams 15 incoming from the upper side of a skin 50 are concentrated onto the subcutaneous target part 51. The holding member 16 has a configuration in which the optical fibers 10 are attached thereto by screws (not shown) or the like so that the concentration position can be changed according to the target part 51.

Actions of the circulation-accelerating laser irradiation system 1 will be described.

When a therapy start signal is inputted by the user or the like, the control unit 13 starts controlling the laser beam generation unit 12. The laser beam generation unit 12 generates the laser beams 15 according to the control of the control unit 13. The laser beams 15 thus generated are transmitted by the optical fibers 10, and are converted into parallel beams by the collimator lenses 11. The laser beams 15 converted into the parallel beams are radiated on the living body, and are concentrated onto the target part 51. The diseased part is treated by the energy of the laser beams 15 thus concentrated.

Here, the wavelength of the laser beams 15 generated by the laser beam generation unit 12 is a wavelength in the visible region; specifically, the wavelength is preferably 400 to 650 nm, particularly 400 to 600 nm. The laser beams 15 from the individual optical fibers 10 may have an equal wavelength or different wavelengths in the wavelength range.

In addition, the output energy is preferably not less than 5 mW per optical fiber 10. If the output energy is less than 5 mW, even where the laser beams 15 are radiated from the plurality of the optical fibers 10, the energy of each individual laser beam 15 is so weak that each individual laser beam 15 does not reach a part under the skin tissue, so that a therapeutic effect at the subcutaneous deep part cannot be expected. On the other hand, the output energy is desirably not more than 1000 mW. If the output energy per optical fiber 10 is in excess of 1000 mW, there arises the fear of bad influences, such as damage to the skin tissue.

In addition, the number of the optical fibers 10 corresponding to the number of outgoing ports of the laser beams 15 varies depending on the output energy per optical fiber 10, is not particularly limited; the number may be appropriately determined according to the purpose of the therapy or to the effect expected. It should be noted, however, that if the number of the optical fibers 10 is too large, when the laser beams 15 from a plurality of semiconductor data devices are concentrated onto the target part 51, the total energy amount of the beams thus concentrated may produce a bad influence on the living body tissue, and, therefore, it is necessary to carefully set the total energy amount at the concentration part to a value of 50 mW or below. As to the upper limit of the output energy of the laser beams 15, sufficient attention must be paid according to the condition of the focus part or to the patient to be treated. Therefore, the above-mentioned values are not values which should necessarily be preferred. These values should naturally be determined while appropriately paying sufficient attention. From this point of view, the laser beam generation unit 12 is preferably so configured that its output can be regulated as required.

Thus, in the first embodiment, the laser beams 15 from the plurality of optical fibers 10 are radiated onto a living body, and are concentrated onto a subcutaneous target part 51 of the living body, whereby a sufficient energy for therapy can be obtained at the target part 51, notwithstanding each individual laser beam 15 has a weak output. Since it suffices for each individual laser beam 15 to have a weak output, it is possible to prevent bad influences of the laser beam 15 from being exerted on the skin tissues irradiated with each individual laser beam 15 and, simultaneously, to enhance the therapeutic effect on the target part 51 (i.e., focus part) through the concentration of the laser beams 15.

In addition, since the concentrated laser beams 15 reach the tissue under the skin 50 notwithstanding the output energy of each laser beam 15 is weak, the concentration of these laser beams 15 promises an effective therapy of a focus part present at a subcutaneous deep part, such as blood vessels in the fascias or muscle present under the subcutaneous fatty tissue in the cases of muscle- or fascias-related lumbago.

Besides, with the plurality of laser beams 15 radiated from many directions and concentrated onto the target part 51, it is possible to suppress the influences of the laser beams 15 on the surroundings of the target part 51, and to specify the part irradiated with the laser beams 15, thereby preventing the laser beams 15 from being concentrated onto other parts.

Although the circulation-accelerating laser irradiation system 1 is used with a focus part at a subcutaneous deep part as the target part 51, it can be expected to be effective also for a peripheral circulation insufficiency present at a comparatively surface layer. Specifically, the circulation-accelerating laser irradiation system 1 is effective for treatment of a wide variety of diseases attendant on circulation inefficiency, such as muscle- or fascias-related lumbago, stiff shoulders, stenocardia, bedsore, asteriosclerosis obliterans (ASO), arteritis obliterans (Buerger's disease TAO), and diabetic arterial obliteration, which are present subcutaneously.

Furthermore, the circulation-accelerating laser irradiation system 1 is considered to be useful also for accelerating the cure of an operative wound, for which a therapeutic effect can be expected to be produced by improvement of bloodstream.

### [Second Embodiment]

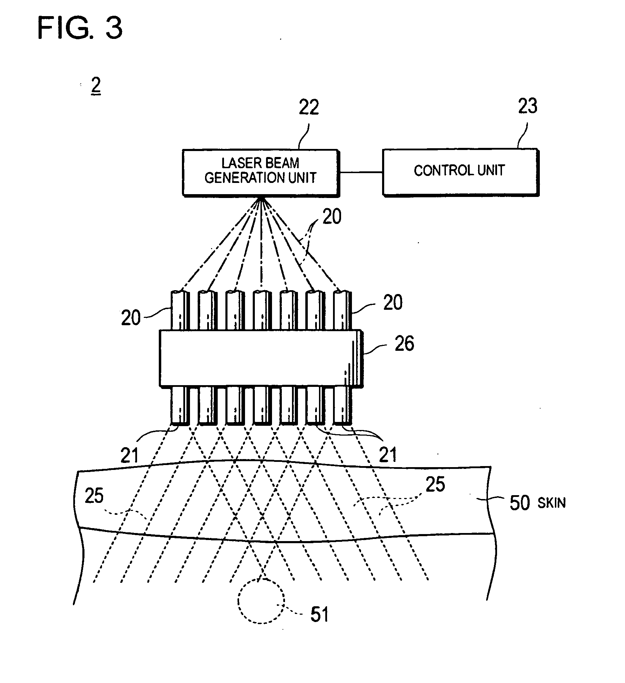

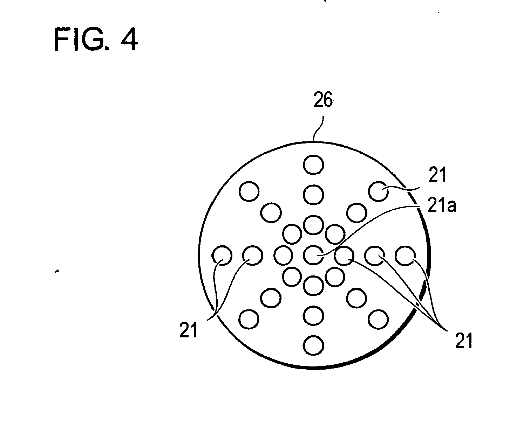

Fig. 3 is a schematic diagram showing a circulation-accelerating laser irradiation system according to a second embodiment of the present invention, and Fig. 4 is a diagram showing outgoing ports of laser beams.

The circulation-accelerating laser irradiation system 2 in the second embodiment includes a plurality of laser irradiation units by which laser beams with such a wavelength as to have a vasodilating action are radiated in a pulsed mode from positions over a skin, a holding member for positioning and fixing laser outgoing ports of the plurality of laser irradiation units in a radial pattern so that the laser beams are concentrated onto a subcutaneous target part, and a control unit for such a control that the irradiations by the plurality of laser irradiation units are conducted at time intervals.

The laser irradiation unit is composed of a laser beam generation unit 22 for generating pulsed laser beams 25, and optical fibers 20 for transmitting the laser beams 25 generated by the laser beam generation unit 22. The laser beam generation unit 22 is connected to the plurality of optical fibers 20, and can supply the laser beams respectively into the optical fibers 20. The laser beam generation unit 22 is connected further to a control unit 23.

The plurality of optical fibers 20 are fixed by a holding member 26. The holding member 26 positions the plurality of optical fibers 20 so that laser beam outgoing ports 21 of the optical fibers 20 are arranged radially and that the laser beams 25 radiated from the optical fibers 20 in a pulsed mode are concentrated onto the target part (focus part) 51 (see Fig. 4). The laser beam 25 outgoing from the outgoing port 21 is not converted into a parallel beam by a collimator lens or the like, so that the laser beam 25 is diffused, as shown in Fig. 3.

However, since the laser beams 25 are diffused from all the outgoing ports 21 at roughly equal angles, as shown in Fig. 3, so that a portion of each laser beam proceeds toward the target part 51. As a result, the laser beams 25 are concentrated into a position under the outgoing port 21a disposed at the center of the radial pattern. Therefore, with the target part 51 disposed on the lower side of the outgoing port 21a, the laser beams 25 enters into the skin 50 from above and are concentrated to the subcutaneous target part 51.

Incidentally, the holding member 26 has a configuration in which the optical fibers 20 are attached thereto by screws (not shown) or the like so that the laser beam concentration position can be conformed to the target part 51 by regulating the protrusion amounts of the optical fibers 20 or the like.

Now, actions of the circulation-accelerating laser beam irradiation system 2 will be described.

When a therapy start signal is inputted by the user or the like, the control unit 13 starts controlling the laser beam generation unit 12. The control unit 13 performs such a control that the laser beams 25 are radiated from the outgoing ports 21 disposed in the radial pattern, at slight time intervals in the order from the outer side toward the inner side, as has been described above. Namely, the laser beams 25 are supplied at slight time intervals in the order from the optical fiber 20 corresponding to the outgoing port 21 on the outer side toward the optical fiber 20 corresponding to the outgoing port 21 on the inner side.

The laser beams 25 supplied outgo at slight timer intervals, in the order from the outgoing port 21 on the outer side toward the outgoing port 21 on the inner side. Since the laser beams 25 outgoing in the order from the outgoing port 21 on the outer side are radiated in the order from the farthest from the target part 51, so that when the laser beams 25 are radiated at slight time intervals, the laser beams 25 reach the target part 51 roughly simultaneously.

Therefore, the target part 51 is irradiated with the laser beams simultaneously, and is supplied with a high overall energy; on the other hand, each of other parts is not irradiated with the laser beams simultaneously, so that it is supplied only with low energy. As a result, at the target part 51, therapy can be conducted by the output of the laser beams 25, whereas at other parts, there is no influence of the output of the laser beams 15, and the normal living body would not be damaged.

Thus, in the second embodiment, the outgoing ports 21 of the optical fibers 20 are arranged in a radial pattern on a plane roughly parallel to the target part 51, and such a control is conducted that the laser beams 25 are radiated in the order from the outer side in the radial pattern, i.e., in the order from the farthest from the target part 51. Therefore, at the target part 51, the laser beams 25 are simultaneously concentrated, so that a high energy can be applied to the target part 51. As a result, at the target part 51, an energy sufficient for therapy can be obtained, and the therapeutic effect can be enhanced through the concentration.

On the other hand, at each of other parts than the target part 51, the laser beams 25 from the outgoing ports 21 would not be concentrated, and simultaneous irradiation with the laser beams 25 would not occur. Therefore, each of the other parts is not supplied with a high energy. As a result, at other parts than the target part 51, a high energy is not applied by the laser beams 25, and the skin tissue can be prevented from being damaged.

In addition to the above-mentioned effect, the same kind of effect as in the first embodiment is also obtained. Here, in the second embodiment, the laser beams 25 are not converted into parallel beams by collimator lenses but are let diffuse, and, at other parts than the target part 51, such a control is conducted that the laser beams 25 from the different outgoing ports 21 would not reach simultaneously. Therefore, the energy supplied by the laser beams 25 is low at other parts than the target part 51, so that the influence exerted on the normal skin tissue is more reduced in the second embodiment.

Incidentally, while an example of irradiating with the laser beams 25 at time intervals in the order from the outer side of the outgoing ports 21 has been described in the second embodiment, this mode is not limitative. For example, the irradiation with the laser beams 25 may be conducted at time intervals in the order from the inner side of the outgoing ports 21. Besides, the irradiation with the laser beams 25 may be conducted at random. In this case, also, the normal skin is not supplied with high energy, and can therefore be prevented from being damaged. In addition, the target part 51 is irradiated with the laser beams 25 not simultaneously but without interruption, so that sufficient energy is supplied only to the diseased part.

In addition, a configuration in which the outgoing ports 21 of the optical fibers 20 are arranged in a radial pattern on a plane roughly parallel to the target part 51 has been described, the arrangement of the outgoing ports 21 is not limited to this configuration. The outgoing ports 21 may be arranged in a grid pattern or in a concentric circle pattern. Irrespectively of the mode of arrangement, by radiating the laser beams 25 in the order from the outer side toward the inner side of the plurality of outgoing ports 21 thus arranged, the laser beams 25 are concentrated on the target part 51 simultaneously, and high energy can be applied to the target part 51.

Besides, while the case where pulsed laser beams are utilized as the laser beams has been described, continuous laser beams can also be used inasmuch as their output is on such a level as not to influence the skin surface.

Further, a high-luminance LED having such a wavelength as to have the above-mentioned vasodilating action or the like can also be used, in place of the laser beam.

### Industrial Applicability

According to the circulation-accelerating laser irradiation system of the present invention, the plurality of laser beams radiated from the plurality of laser irradiation means are concentrated onto a subcutaneous target part, so that a sufficient energy for therapy can be obtained at the target part notwithstanding the laser beam from each individual laser irradiation means has a weak output. Since each individual laser beam may be weak in output, it is possible to prevent the skin tissue irradiated with each individual laser beam from being badly influenced by the laser beam, and, simultaneously, to enhance the therapeutic effect on the target part (i.e., focus part) through the concentration of the laser beams.

Besides, according to the another circulation-accelerating laser irradiation system, the radiation ports of the laser irradiation means are positioned in a radial pattern, and the plurality of laser irradiation means are so controlled that the irradiation with the laser beams occur at time intervals. Therefore, at the target part, the laser beams are concentrated simultaneously to apply high energy to the target part. As a result, at the target part, a sufficient energy for therapy can be obtained, and the therapeutic effect can be enhanced through the concentration of the laser beams.

On the other hand, at other parts than the target part, the laser beams from the outgoing ports are not concentrated, and simultaneous irradiation with the laser beams does not occur, so that high energy is not applied. As a result, at other parts than the target part, application of high energy by the laser beams does not occur, and the skin tissue can be prevented from being damaged.

## Claims

1. A blood circulation-accelerating laser irradiation system (2) comprising:
a plurality of laser irradiation sections by which laser beams with wavelengths of 400 to 650 nm are radiated as pulses from positions over a skin;
a holding section (26) for positioning and fixing laser beam outgoing ports (21) of said plurality of laser irradiation sections; and
a control section (23) for such a control that laser irradiations by said plurality of laser irradiation section are conducted at time intervals,
wherein said laser irradiation sections are positioned and fixed in a radial pattern so that said laser beams are concentrated onto a subcutaneous target part (51),
**characterized in that** said control section (23) performs such a control that irradiations with laser beams from said outgoing ports (21) arranged in said radial pattern are conducted at time intervals in the order
from the outer side of the plurality of the outgoing ports (21) toward the inner side of the plurality of the outgoing ports (21) towards said subcutaneous target part (51) or from the inner side toward the outer side of the plurality of the outgoing ports (21) away from said subcutaneous target part (51),
wherein the irradiations are conducted so that the laser beams reach said subcutaneous target part (51) substantially simultaneously.

2. The blood circulation-accelerating laser irradiation system as set forth in claim 1, wherein said plurality of laser irradiation sections each comprise:
a laser beam generating section (22) for generating the laser beam; and
an optical fiber (20) for transmitting said laser beam generated by said laser beam generating section.

3. The blood circulation-accelerating laser irradiation system as set forth in claim 1 or 2, wherein the optical energy radiated from one of said laser irradiation section is not less than 5 mW.

## Patentansprüche

1. Laserbestrahlungssystem (2) zur Beschleunigung des Blutkreislaufs, umfassend:
eine Vielzahl von Laserbestrahlungsabschnitten, durch die Laserstrahlen mit Wellenlängen von 400 bis 650 nm als Impulse von Positionen über einer Haut abgestrahlt werden;
einen Halteabschnitt (26) zum Positionieren und Fixieren von Laserstrahl-Ausgangsöffnungen (21) der Vielzahl von Laserbestrahlungsabschnitten; und
einen Steuerabschnitt (23) für eine solche Steuerung, dass Laserbestrahlungen durch die Vielzahl von Laserbestrahlungsabschnitten in Zeitabständen durchgeführt werden,
wobei die Laserbestrahlungsabschnitte in einem radialen Muster positioniert und fixiert sind, so dass die Laserstrahlen auf einen subkutanen Zielbereich (51) konzentriert sind,
**dadurch gekennzeichnet, dass** der Steuerabschnitt (23) eine solche Steuerung durchführt, dass Bestrahlungen mit Laserstrahlen von den in dem radialen Muster angeordneten Ausgangsöffnungen (21) in Zeitabständen von der Außenseite der Vielzahl von Ausgangsöffnungen (21) in Richtung zur Innenseite der Vielzahl von Ausgangsöffnungen (21) in Richtung zu dem subkutanen Zielbereich (51) oder von der Innenseite in Richtung zur Außenseite der Vielzahl von Ausgangsöffnungen (21) weg von dem subkutanen Zielbereich (51) durchgeführt werden,
wobei die Bestrahlungen so durchgeführt werden, dass die Laserstrahlen den subkutanen Zielbereich (51) im Wesentlichen gleichzeitig erreichen.

2. Laserbestrahlungssystem zur Beschleunigung des Blutkreislaufs nach Anspruch 1, wobei die Vielzahl von Laserbestrahlungsabschnitten jeweils Folgendes umfassen:
einen Laserstrahlerzeugungsabschnitt (22) zum Erzeugen des Laserstrahls; und
eine optische Faser (20) zum Übertragen des durch den Laserstrahlerzeugungsabschnitt erzeugten Laserstrahls.

3. Laserbestrahlungssystem zur Beschleunigung des Blutkreislaufs nach Anspruch 1 oder 2, wobei die von einem der Laserbestrahlungsabschnitte abgestrahlte optische Energie nicht weniger als 5 mW beträgt.

## Revendications

1. Système de rayonnement laser (2) pour accélérer la circulation sanguine comprenant :
une pluralité de sections de rayonnement laser par lesquelles des faisceaux laser ayant des longueurs d'onde de 400 à 650 nm sont émis par impulsions à partir de positions situées au-dessus d'une peau ;
une section de support (26) pour positionner et fixer des orifices de sortie de faisceau laser (21) de ladite pluralité de sections de rayonnement laser ; et
une section de commande (23) destinée à commander la pluralité de sections de rayonnement laser de manière telle que les rayonnement laser sont émis par intervalles de temps,
dans lequel lesdites sections de rayonnement laser sont positionnées et fixées selon un motif radial de telle manière que lesdits faisceaux laser sont concentrés sur une partie de cible sous-cutanée (51),
**caractérisé en ce que** ladite section de commande (23) effectue une commande telle que les rayonnements des faisceaux laser provenant des orifices de sortie (21) agencés selon ledit motif radial sont émis par intervalles de temps dans l'ordre allant du côté extérieur de la pluralité d'orifices de sortie (21) vers le côté intérieur de la pluralité d'orifices de sortie (21) en direction de ladite partie de cible sous-cutanée (51) ou du côté intérieur vers le côté extérieur de la pluralité d'orifices de sortie (21) en s'éloignant de ladite partie de cible sous-cutanée (51),
dans lequel les rayonnements sont émis de telle manière que les faisceaux laser atteignent ladite partie de cible sous-cutanée (51) de façon sensiblement simultanée.

2. Système de rayonnement laser pour accélérer la circulation sanguine selon la revendication 1, dans lequel lesdites sections de rayonnement laser comprennent chacune :
une section de génération de faisceau laser (22) pour générer le faisceau laser ; et
une fibre optique (20) pour transmettre ledit faisceau laser généré par ladite section de génération de faisceau laser.

3. Système de rayonnement laser pour accélérer la circulation sanguine selon la revendication 1 ou 2, dans lequel l'énergie optique émise par l'une desdites sections de rayonnement laser est supérieure ou égale à 5 mW.
